# DEMANDE DE BREVET EUROPEEN

(11) **EP 0 724 016 A1**
(43) Date de publication de la demande: **31.07.1996**
(21) Numéro de dépôt: 96420030.7
(22) Date de dépôt: 29.01.1996
(51) Int. Cl.: C12N 15/12, C12N 15/63, C07K 14/45, G01N 33/569, A61K 39/002

(54) **Polypeptides mimotopes de Toxoplasma gondii et applications**

(30) Priorité: 30.01.1995 FR 9501297
(71) Demandeur: BIO MERIEUX, F-69280 Marcy l'Etoile (FR)
(72) Inventeur: Jolivet-Reynaud, Colette, F-69500 Bron (FR)
(74) Mandataire: Guerre, Dominique

(57) **Abrégé**

L'invention concerne un polypeptide capable de réagir spécifiquement avec un anticorps anti protéine P30 de *Toxoplasma gondii*, et comprenant une séquence peptidique, dont toute suite de 6 acides aminés contigus présente au plus 67 % d'homologie avec la séquence peptidique de ladite protéine P30, identifiée par SEQ ID N01, ou comprenant une séquence dérivée de ladite séquence peptidique, et les applications de ce polypeptide notamment pour détecter une infection par *Toxoplasma gondii*, dans un échantillon biologique.

## Description

La présente invention concerne un polypeptide, capable de se substituer à un antigène de *Toxoplasma gondii,* un polynucléotide dont l'expression correspond audit polypeptide, et leur utilisation à des fins diagnostiques et thérapeutiques.

La toxoplasmose est une maladie infectieuse causée par un parasite protozoaire *Toxoplasma gondii,* membre de la classe des Sporozoa et de l'ordre des Coccidia. *Toxoplasma gondii* est un parasite intracellulaire qui se reproduit dans une grande variété de types cellulaires chez ses hôtes, qui sont les mammifères.

Ce parasite, qui est très largement répandu géographiquement, est un agent pathogène important, non seulement en médecine humaine, mais également en médecine vétérinaire.

Chez l'homme, deux formes du parasite ont été décrites : le "tachyzoïte", qui est la forme multiplicative rencontrée au cours de la phase aigüe de la maladie et le "bradyzoïte", forme résistante qui persiste enkystée dans les tissus nerveux, et qui est vraisemblablement responsable de l'entretien d'une immunité durable à la réinfection.

Chez l'être humain, la toxoplasmose est le plus souvent asymptomatique et passe la plupart du temps inaperçue sans présenter de conséquences. Il existe cependant des cas pour lesquels une infection par le toxoplasme ou une réactivation d'une infection acquise antérieurement peut générer des troubles graves pour les personnes dites à risque que sont les femmes enceintes et les sujets immunodéprimés ou immunosupprimés. Cet organisme a des sièges de réplication multiples. Ainsi, il peut être responsable d'atteintes oculaires et cérébrales sévères lorsque son siège de réplication est les cellules du système nerveux central et les cellules du système réticuloendothélial. La femme enceinte représente un sujet à haut risque, puisqu'une infection toxoplasmique, notamment pendant les premiers mois de la grossesse, peut être à l'origine de graves complications foetales et néonatales si le traitement maternel n'est pas précocement entrepris et poursuivi avec assiduité. En particulier, les nouveaux nés contaminés par voie transplacentaire sont sujets à des troubles cérébraux et oculaires graves, voire mortels dans certains cas. Les malades immunodéprimés et particulièrement les sidéens sont sujets à des toxoplasmoses graves dues le plus souvent à des réactivations d'infections antérieures, bien qu'une dissémination suite à une primo-infection ne puisse être totalement exclue (voir FARKASH et al., J. Neurology, Neurosurgery and Psychiatry 1986, 49, 744-748 et Luft et al, J. Am. Med. Ass. 1984, 252, 913-917).

Il est donc indispensable de disposer de tests de diagnostic qui permettent de déterminer la présence du parasite, notamment chez la femme enceinte, soit par détection d'anticorps spécifiques, soit par détection d'antigènes du toxoplasme chez le sujet.

HUGUES dans "Current topics in Microbiology and Immunology" (Vol.120, 1985, SPRINGER Ed., pages 105-139) a répertorié un certain nombre de tests de sérodiagnotic disponibles commercialement, tels que le test de coloration de SABIN et FELDMAN, standardisé par BEVERLY et BEATTLE en 1958 et perfectionné par FELDMAN et LAMB (1966), WALDELAND (1976) et BALFOUR et al. (1982) ; le test de détection d'anticorps par immunofluorescence de REMINGTON (1968), optimisé en 1975 par KARIM et LUDLAM ; les tests d'hémagglutination ; le test ELISA pour la détection d'anticorps spécifique pour le Toxoplasme, par isolement d'IgM in situ sur microplaque décrit en 1983 par WIELARRD et al.

Les différents tests mis en oeuvre reposent sur la formation de complexes immuns entre les antigènes de *Toxoplasma gondii* et leurs anticorps spécifiques. Un des points critiques consiste donc en la caractérisation des antigènes majeurs de *Toxoplasma gondii,* qui induisent une réponse immunitaire spécifique et sont susceptibles d'être utilisés dans des tests sérologiques de détection.

Des auteurs ont montré que la plupart des anticorps monoclonaux dirigés contre la surface de *Toxoplasma gondii* reconnaissent une protéine de 27 à 35 KDa, nommée P30 (Kasper et al.; 1983, J. Immunol. 130, 2407-2412). Plusieurs travaux ont mis en évidence que cette protéine P30 est un antigène majeur de surface qui peut être utilisé pour la production de vaccins ou dans des tests de diagnostic, notamment dans des immunoessais. Par ailleurs, Boothroyd et al.(voir la demande de brevet WO 89/08700) ont identifié et obtenu le matériel génétique codant pour la protéine P30 de *Toxoplasma gondii* et suggèrent l'utilisation du gène pour la production de protéine recombinante, de peptides et d'anticorps. Ce gène a été cloné (Burg et al., 1988, J. Immunol., 141, 3584-3591). L'analyse de la séquence montre un site potentiel de N-glycosylation, un signal de sécrétion positionné à l'extrémité N-terminale qui est clivé dans la protéine P30 mature et une région C-terminale fortement hydrophobe également clivée et remplacée par un glycolipide permettant l'ancrage membranaire de la protéine P30.

Il subsiste cependant un problème qui consiste en l'obtention de quantités suffisantes de l'antigène P30. En effet, dans le but d'obtenir des antigènes ou des parties d'antigènes P30, utilisables notamment dans des procédés de diagnostic, trois approches sont actuellement disponibles.

La première consiste à réaliser des cultures du parasite *Toxoplasma gondii* dans un grand nombre de souris infectées, à prélever les antigènes naturels de *Toxoplasma gondii,* et notamment la P30, par voie péritonéale et à les purifier. Toutefois, cette technique présente de nombreux inconvénients tels que le coût, la nécessité de disposer d'un personnel expérimenté et d'un grand nombre d'animaux, la complexité de la technique ou les difficultés liées à l'extraction qui est peu performante.

La seconde approche, dérivée de la précédente, fait appel à la culture *in vitro* du parasite dans des cultures cellulaires (Grimwood et al. 1979, Experimental Parasitology, 48, 282-286). Toutefois, cette approche ne permet pas de pallier les inconvénients de coût ou d'extraction précités.

La troisième approche fait référence aux technologies de recombinaison génétique et consiste à introduire et cloner dans un génome procaryote, eucaryote ou viral, tout ou partie du gène codant pour la protéine P30, à exprimer ledit gène dans une cellule hôte et à extraire la protéine P30 recombinante obtenue. Cette technique offre de nombreux avantages mais souffre d'un faible taux d'expression de la protéine. Par ailleurs, Burg et al. (1988, Journal of Immunology, 141, 3584-3591) ont montré que la protéine P30 naturelle est un antigène fortement structuré présentant des contraintes conformationnelles. La conformation de la protéine P30 est un élément déterminant pour sa reconnaissance par des anticorps spécifiques et résulte notamment de la présence de ponts disulfure dans la protéine. Or, il est difficile, par recombinaison génétique, de maitriser parfaitement les modifications post-traductionnelles que subissent les protéines synthétisées. Par conséquent, il est difficile d'obtenir, par recombinaison génétique, une protéine P30 ou une partie de ladite protéine qui soit convenablement maturée de manière à ce qu'elle adopte une conformation suffisamment proche de celle de la protéine P30 naturelle pour permettre une reconnaissance spécifique par les anticorps dirigés contre ladite protéine.

Conformément au document WO-A-89/12683, il est décrit des polypeptides capables de réagir avec un anticorps anti-protéine P30 de *Toxoplasma gondii* et possédant une séquence peptidique choisie parmi la séquence SEQ ID N015, les fragments de SEQ ID N015, SEQ ID N016, SEQ ID N017 et SEQ ID N018, identifiées en fin de description. Ces polypeptides ont été déterminés à partir de la séquence peptidique d'une protéine de *Toxoplasma gondii,* et consistent en la séquence de cette dernière ou des fragments antigéniques de cette dernière. Etant donné le faible taux d'homologie desdites séquences avec celle de la protéine P30, on peut avancer que la séquence peptidique de la protéine à l'origine de ces polypeptides n'est pas celle de la protéine P30. Ces polypeptides posent le problème de leur spécificité ; en effet ils ont des propriétés antigéniques vis-à-vis des anticorps anti-P30, mais ils ont probablement les mêmes propriétés vis-à-vis des anticorps dirigés contre la protéine de laquelle ils ont été définis.

De façon surprenante, on a montré qu'un polypeptide synthétisé par voie chimique ou par le biais des méthodologies de recombinaison génétique, n'ayant subi aucune modification après sa synthèse, est capable d'être reconnu par des anticorps spécifiques de la protéine P30, notés anti-P30.

Avant d'aborder les objets de l'invention, différents termes employés dans la description qui va suivre sont ci-après définis.

Par "protéine P30", on entend l'antigène majeur de *Toxoplasma gondii,* donc la séquence peptidique est identifiée par la référence SEQ ID N01.

Par "sera d'individus ou d'animaux infectés par *Toxoplasma gondii,"* on entend notamment des sera de patients ayant contracté une infection récente ou ancienne par *Toxoplasma gondii* et qui contiennent des immunoglobulines reconnaissant spécifiquement tout ou partie de la protéine P30.

Bien entendu, un polypeptide selon l'invention peut également être reconnu par d'autres anticorps, comme par exemple des anticorps monoclonaux ou polyclonaux obtenus par immunisation d'espèces variées par la protéine P30 naturelle, la protéine P30 recombinante, leurs fragments ou peptides.

Par "polypeptide", on désigne un peptide ou un fragment peptidique, ou une protéine, obtenu par synthèse chimique ou par des techniques de recombinaison génétique. Les polypeptides selon l'invention peuvent être obtenus par des méthodes de synthèse classique, par exemple dans un synthétiseur automatique de peptides, ou par les techniques de génie génétique comprenant l'insertion d'une séquence d'ADN codant pour ledit polypeptide dans un vecteur d'expression tel qu'un plasmide ou un virus comme décrit dans la présente demande, et la transformation de cellules avec ce vecteur d'expression et culture de ces cellules eucaryotes ou procaryotes.

Le degré d'homologie d'une première séquence peptidique, par rapport à une deuxième séquence peptidique de référence, caractérise le degré maximum, exprimé en pourcentage, d'identité entre les deux séquences. Il est évalué, pour une suite d'un nombre donné d'acides aminés contigus, par alignement des deux séquences, et en déplaçant l'une par rapport à l'autre, et en comparant les acides aminés dans les deux séquences.

Par "séquence dérivée d'une autre séquence d'acides aminés", on entend une séquence d'acides aminés modifiée par insertion et/ou délétion et/ou substitution et/ou allongement et/ou raccourcissement et/ou modification chimique d'un ou plusieurs acides aminés, à l'exclusion de toute séquence peptidique dont toute suite de 20 acides aminés contigus présente au moins 40% d'homologie avec la séquence peptidique de la protéine P30 (SEQ ID NO1), pour autant que ces modifications préservent substantiellement voire développent les propriétés de ladite séquence. Ainsi on entend par "séquence dérivée", notamment les séquences dans lesquelles un ou plusieurs acide aminé est substitué par un ou plusieurs autres acides aminés comme illustré à l'Exemple 6 ; les séquences dans lesquelles un ou plusieurs acide aminé de la série L est remplacé par un acide aminé de la série D, et vice-versa ; les séquences dans lesquelles on a introduit une modification des chaines latérales des acides aminés, telle que par exemple une acétylation des fonctions amines, une carboxylation des fonctions thiols, une estérification des fonctions carboxyliques ; une modification des liaisons peptidiques telles que par exemple des liaisons carba, retro, inverso, retro-inverso, réduites et méthylène-oxy.

Par "polynucléotide", on entend soit une séquence d'ADN, soit une séquence d'ARN, soit une séquence d'ADNc résultant de la transcription inverse d'une séquence d'ARN, d'origine naturelle ou de synthèse, avec ou sans bases modifiées.

La présente invention a pour objet un polypeptide capable de réagir spécifiquement avec un anticorps anti-protéine P30 de *Toxoplasma gondii,* et comprenant une séquence peptidique, présentant, dans toute suite de 6 acides aminés contigus, au plus 67 % d'homologie avec la séquence peptidique de ladite protéine P30, identifiée par SEQ ID N01, ou comprenant une séquence dérivée de ladite séquence peptidique.

De manière préférentielle, un polypeptide de l'invention comprend une séquence peptidique, présentant, dans toute suite de 11 acides aminés contigus, au plus 36 % d'homologie avec ladite séquence peptidique SEQ ID N01, ou comprend une séquence dérivée. Un polypeptide particulier de l'invention possède une séquence peptidique consistant en une séquence peptidique, présentant, dans toute suite de 11 acides aminés contigus, au plus 36 % d'homologie avec ladite séquence peptidique SEQ ID N01, ou consistant en une séquence dérivée.

La présente invention a également pour objet un polypeptide capable de réagir spécifiquement avec un anticorps anti-protéine P30 de *Toxoplasma gondii,* qui possède une séquence peptidique comprenant SEQ ID N02, ou une séquence dérivée.

La séquence peptidique d'un tel polypeptide comprend avantageusement une séquence choisie parmi SEQ ID N03, SEQ ID N04, et leurs séquences respectivement dérivées.

En particulier, ladite séquence peptidique comprend une séquence choisie parmi SEQ ID N05, SEQ ID N06, et leurs séquences respectivement dérivées.

De manière encore préférentielle, ladite séquence peptidique comprend une séquence choisie parmi SEQ ID N07, SEQ ID N08, SEQ ID N09, SEQ ID N010, et leurs séquences respectivement dérivées.

Un polypeptide particulier de l'invention possède une séquence peptidique consistant en une séquence choisie parmi SEQ ID N02, SEQ ID N03, SEQ ID N04, SEQ ID N05, SEQ ID N06, SEQ ID N07, SEQ ID N08, SEQ ID N09, SEQ ID N010, et leurs séquences respectivement dérivées.

Le polypetide tel que défini précédemment peut être d'origine phagique ou virale ou bactérienne ou eucaryote inférieur ou eucaryote supérieur.

En particulier, ce polypeptide peut être un fragment peptidique d'enveloppe, un fragment peptidique membranaire ou un fragment peptidique susceptible d'être excrété.

Par ailleurs, lesdits polypeptides peuvent être conjuqués selon des techniques bien connues de l'homme du métier, à une molécule porteuse comme par exemple une protéine naturelle ou recombinante (exception faite de la protéine P30 naturelle ou recombinante), un polymère synthétique d'acides aminés ou de chaînes aliphatiques, un fragment nucléique ou à une molécule traceuse comme par exemple un oligonucléotide, une enzyme telle que notamment la peroxydase de raifort, la phosphatase alcaline ou la galactosidase ou encore un radioélément ou fixés sur tout support.

Il est bien entendu que toute séquence peptidique différente de celles décrites dans l'invention, et suceptible d'être comprise dans la séquence peptidique d'un polypeptide de l'invention ne doit pas, en tant que telle, être capable de réagir avec un anticorps anti-protéine P30.

Un second objet de la présente invention est une application d'un polypeptide décrit ci-dessus et consiste en un réactif pour la détection d'une infection par *Toxoplasma gondii,* ledit réactif comprenant, à titre de substance réactive, un polypeptide de l'invention.

Un troisième objet de l'invention est un kit de détection d'une infection par *Toxoplama gondii,* comprenant le réactif décrit ci-dessus, supporté par un support solide, immunologiquement compatible avec ledit réactif.

Le terme support solide tel qu'utilisé ici est sans limitation sous la forme d'une plaque de microtitration, d'une feuille, d'un cône, d'un puits, d'une bille, ou tout autre substrat micro-particulaire approprié, et inclut tous les matériaux sur lesquels peuvent être immobilisés les fragments peptidiques de l'invention. Ce peut être des matériaux de synthèse modifiés ou non chimiquement, notamment des polysaccharides, tels que les matériaux de cellulose, par exemple du papier, des dérivés de cellulose tels que la nitrocellulose et l'acétate de cellulose; des polymères tels que chlorure de vinyle, polyéthylène, polystyrène, polyacrylate, ou copolymères tels que polymère de chlorure de vinyle et de propylène, polymère de chlorure de vinyle et d'acétate de vinyle; copolymères à base de styrène; des fibres naturelles telles que le coton et des fibres synthétiques telles que le nylon. De préférence, le support solide est un polymère de polystyrène, un copolymère butadiène-styrène ou un copolymère butadiène-styrène en mélange avec un ou plusieurs polymères ou copolymères choisis parmi le polystyrène, les copolymères styrène-acrylonitrile ou styrène-méthylméthacrylate de méthyle, les polypropylènes, les polycarbonates ou analogues.

La fixation du réactif sur le support solide peut être réalisée de manière directe ou indirecte.

De manière directe, deux approches sont possibles: soit par adsorption du réactif sur le support solide, c'est à dire par des liaisons non covalentes (principalement de type hydrogène, Van der Wals ou ionique), soit par établissement de liaisons covalentes entre le réactif et le support. De manière indirecte, on peut fixer préalablement (par adsorption ou covalence) sur le support solide un composé "anti-réactif" capable d'interagir avec le réactif de façon à immobiliser l'ensemble sur le support solide.

L'invention apporte en outre un procédé pour la détection et/ou la séparation, et notamment purification, et/ou le dosage d'anticorps anti-protéine P30 dans un échantillon de fluide biologique comprenant les étapes suivantes: on met en contact ledit échantillon avec un réactif de l'invention, dans des conditions permettant une réaction immunologique, puis on détecte, sépare et/ou quantifie le complexe immun éventuellement formé.

Selon une variante, l'invention apporte un procédé pour le dosage de la protéine P30 de *Toxoplasma gondii* dans un échantillon d'un fluide biologique, effectué par une technique de compétition, au cours duquel on met simultanément en contact ledit échantillon avec une quantité prédéterminée d'anticorps anti-protéine P30, et une quantité prédéterminée d'un réactif de l'invention, et on détermine la quantité de protéine P30 dans ledit échantillon par déduction à partir de la quantité mésurée du complexe formé entre le réactif et lesdits anticorps anti-protéine P30.

Selon une autre variante d'un procédé de l'invention pour le dosage de la protéine P30 dans un échantillon d'un fluide biologique, dans un premier temps, on met en contact ledit échantillon avec une quantité prédéterminée d'anticorps anti-protéine P30, dans un second temps, on ajoute une quantité prédéterminée d'un réactif de l'invention, et on détermine la quantité de protéine P30 dans ledit échantillon par déduction à partir de la quantité mésurée du complexe formé entre le réactif et lesdits anticorps anti-protéine P30.

Ces procédés peuvent être basés sur une méthode radioimmunologique de type RIA, RIPA ou IRMA ou une méthode immunoenzymatique de type Western-blot ou ELISA.

Une autre application des polypeptides de l'invention est une composition immunothérapeutique active, notamment préparation vaccinale, comprenant, à titre de principe actif, un polypeptide décrit ci-dessus, ledit principe actif étant éventuellement conjugué avec un support immunologiquement adapté, et éventuellement un excipient pharmaceutiquement acceptable.

L'invention apporte en outre un polynucléotide codant pour un polypeptide décrit ci-dessus. Ce polynucléotide comprend une séquence nucléotidique notamment choisie parmi SEQ ID N0 11, SEQ ID N012, SEQ ID N013, SEQ ID N014.

Elle concerne aussi une cassette d'expression fonctionnelle permettant l'expression d'un polynucléotide de l'invention et comprenant ce dernier, ainsi qu'un vecteur et un système cellulaire eucaryote ou procaryote comprenant une cassette d'expression.

La présente invention est maintenant illustrée à partir des exemples 1 à 7 et des dessins dans lesquels:

La figure 1 illustre l'inhibition par la protéine P30 native de la réponse ELISA obtenue avec le clone phagique identifié dans l'exemple 2. En abscisse sont indiquées les concentrations, exprimées en µg/ml, de la protéine P30 préincubée avec 6nM d'anticorps monoclonal anti-P30 et en ordonnée les valeurs de densité optique mesurées à 492 nm.

La figure 2 illustre, selon courbe a, l'inhibition par un sérum de patient infecté par *Toxoplasma gondii* de la réponse ELISA obtenue avec le clone phagique identifié dans l'exemple 2, par rapport à un sérum témoin non infecté, selon courbe b. En abscisse sont indiquées les dilutions dudit sérum et en ordonnée les valeurs de densité optique mesurées à 492 nm.

La figure 3 est un histogramme illustrant la réponse en ELISA des pentadécapeptides chevauchants synthétisés selon la technique Pepscan suivants :

(1) FYSHSADGAWHWRHR
(2) YSHSADGAWHWRHRI
(3) SHSADGAWHWRHRIP
(4) HSADGAWHWRHRIPL
(5) SADGAWHWRHRIPLQ
(6) ADGAWHWRHRIPLQL
(7) DGAWHWRHRIPLQLA
(8) GAWHWRHRIPLQLAA
(9) AWHWRHRIPLQLAAG
(10) WHWRHRIPLQLAAGR
(11) HWRHRIPLQLAAGRG
(12) WRHRIPLQLAAGRGA
(13) RHRIPLQLAAGRGAA
(14) HRIPLQLAAGRGAAG
(15) RIPLQLAAGRGAAGA
(16) IPLQLAAGRGAAGAE
(17) PLQLAAGRGAAGAET
(18) LQLAAGRGAAGAETV
(19) QLAAGRGAAGAETVE
(20) LAAGRGAAGAETVES
(21) AAGRGAAGAETVESC
(22) AGRGAAGAETVESCL

Les figures 4A à 4F représentent les réponses en ELISA de différents mutants de substitution du peptide His-Arg-Ile-Pro-Leu-Gln-Leu-Ala-Ala-Gly-Arg-Gly-Ala-Ala-Gly qui sont les suivants:
A : substitution de l'histidine en position 1 X-Arg-Ile-Pro-Leu-Gln-Leu-Ala-Ala-Gly-Arg-Gly-Ala-Ala-Gly
B : substitution de l'arginine en position 2 His-X-Ile-Pro-Leu-Gln-Leu-Ala-Ala-Gly-Arg-Gly-Ala-Ala-Gly
C : substitution de la proline en position 4 His-Arg-Ile-X-Leu-Gln-Leu-Ala-Ala-Gly-Arg-Gly-Ala-Ala-Gly
D : substitution de l'isoleucine en position 3 His-Arg-X-Pro-Leu-Gln-Leu-Ala-Ala-Gly-Arg-Gly-Ala-Ala-Gly
E : substitution de la leucine en position 7 His-Arg-Ile-Pro-Leu-Gln-X-Ala-Ala-Gly-Arg-Gly-Ala-Ala-Gly
F : substitution de la glutamine en position 6

His-Arg-Ile-Pro-Leu-X-Leu-Ala-Ala-Gly-Arg-Gly-Ala-Ala-Gly

L'étoile désigne la réponse obtenue avec le peptide sauvage.

En abscisses, sont représentés les acides aminés selon leur nomenclature à une lettre, en ordonnées sont indiquées les valeurs de densité optique mesurées à 405 nm.

L'invention sera mieux comprise à l'aide des exemples qui suivent. Il doit être bien entendu, toutefois, que ces exemples sont donnés uniquement à titre d'illustration de l'objet de l'invention, dont ils ne constituent en aucune manière une limitation.

### Exemple 1 : Sélection d'un pentadécapeptide capable de réagir spécifiquement avec un anticorps monoclonal anti-P30.

Dans un premier temps, une banque d'expression de pentadécapeptides a été construite dans un phage filamenteux selon la méthode décrite par SCOTT et SMITH (1990, Science, 249, 386-390). Cette banque est réalisée en insérant un oligonucléotide synthétique au niveau d'un gène codant pour une protéine phagique d'enveloppe (protéine PIII) dont cinq copies sont présentes à la surface du phage. Cet oligonucléotide consiste en une séquence ayant un code dégénéré [(NNK)¹⁵] où NNK représente un mélange égal des codons correspondants aux 20 acides aminés et le codon stop Amber. Cette banque d'expression permet d'obtenir à la surface du phage, cinq copies d'une protéine fusionnée (PIII - pentadécapeptide). Le site d'insertion du pentadécapeptide dans la séquence de la protéine PIII correspond à la séquence : NH₂-Ala-Asp-Gly-Ala-[pentadécapeptide]-Gly-Ala-Ala-Gly-Ala-Glu-Thr-Val-Glu-COOH.

Dans un second temps, le fond de boites de Pétri de 35 mm de diamètre est traité avec 1 ml d'une solution de streptavidine à la concentration de 10 µg/ml dans du NaHCO₃ 0,1M et incubé pendant une nuit à 4°C. Après élimination de la solution de streptavidine, une solution de 0,1M NaHCO₃, 0,1% de sérum albumine bovine (BSA), 0,1 µg/ml de streptavidine et 0,02% de NaN₃ est ajoutée afin de saturer les sites de liaison non spécifiques et l'ensemble est incubé pendant 2 heures à température ambiante. Les boites de Pétri sont lavées 6 fois avec du tampon TBS (tampon Tris 0,1M pH7,2) /Tween 0,5% et 10 µg d'anticorps monoclonal anti-P30 1E1E7 (BioMérieux) biotinylé sont ensuite incubés une nuit à 4°C dans les boites de Pétri traitées comme décrit ci-dessus. Ceci permet d'obtenir, de façon connue, des boites de Pétri au fond desquelles sont immobilisés lesdits anticorps par l'intermédiaire de la streptavidine.

Un échantillon de la banque d'expression contenant environ 10¹² virions est alors incubé pendant quatre heures à 4°C en présence desdits anticorps biotinylés fixés au fond de la boite de Pétri. Après plusieurs lavages avec du TBS, les phages qui sont restés fixés aux anticorps anti-P30 sont élués avec 400 µl d'une solution d'HCl 0,1N pH 2,2 contenant 0,1% de BSA, puis neutralisés par 75 µl d'une solution Tris-HCl 1M pH 9,1.

Après concentration à 100µl, la suspension de phages élués est soumise à une étape d'amplification par infection d'une suspension à 5x10⁹ bactéries/ml d'une souche d' *E. coli* (K91Kan). Les bactéries infectées (voir Sambrook et al., 1989. Molecular Cloning : A Laboratory Manual, 2nd Edition, Cold Spring Harbor Laboratory, Cold Spring Harbor) sont incubées pendant 45 minutes à 37°C dans 20ml de milieu NZY (tryptone 10g/l, extrait de levure 5g/l, NaCl 5g/l, pH ajusté à 7) contenant 0,2µg/ml de tétracycline. La concentration en tétracycline du milieu est ensuite élevée à 20mg/ml. Les phages infectieux portant un gène de résistance à la tétracycline, seules les bactéries ayant été infectées par le phage sont amplifiées. La culture des bactéries se poursuit pendant une nuit à 37°C. Après centrifugation de la culture afin d'éliminer les cellules bactériennes, 3 ml de polyéthylène-glycol (PEG) 16,3% - NaCl 3,3M sont ajoutés au surnageant afin de précipiter les phages présents. Après une incubation une nuit à 4°C et une centrifugation, le culot de phages est repris dans 1 ml de TBS (tampon Tris 0,1M pH7,2) et reprécipité par 150 µl de PEG/NaCl. Une centrifugation permet d'obtenir un culot de phages qui est remis en suspension dans 200 µl de TBS. La concentration en phages après cette amplification est d'environ 2x10¹³ virions/ml.

Environ 10¹² virions issus de l'étape d'amplification précédente sont incubés avec une solution d'anticorps monoclonal 1E1E7 100 nM, une nuit à 4°C. Ce mélange est ensuite placé dans une boite de Pétri préalablement traitée à la streptavidine et incubé pendant dix minutes à température ambiante. La boite de Pétri est lavée plusieurs fois avec du TBS (tampon Tris 0,1M pH 7,2) / Tween 0,5% et les phages restés fixés aux anticorps monoclonaux anti-P30 sont élués comme décrit précédemment.

Après une nouvelle phase d'amplification de phages à partir de 100 µl de phages élués et une nouvelle phase de sélection par les anticorps monoclonaux anti-P30 1E1E7 (à la concentration de 0,1 nM), 58 colonies bactériennes infectées sont isolées et 30 choisies au hasard sont inoculées individuellement dans 1,7 ml de milieu NZY-tétracycline (20 µg/ml). Après culture sous agitation pendant 16 à 24 heures à 37°C, les cellules sont éliminées par centrifugation. Le surnageant (lml) est mélangé à 150 µl d'une solution de PEG/NaCl et incubé pendant quatre heures à 4°C. Après centrifugation, les phages sont remis en suspension dans 500 µl de TBS.

Les préparation de phages ainsi obtenues contiennent environ 5x10¹¹ phages par millilitre.

Cette sélection de phages à partir d'une banque d'expression a été réalisée par un anticorps monoclonal spécifique de la protéine P30. Par ailleurs, il a été vérifié que les phages, dans lesquels aucun oligonucléotide n'a été inséré, ne réagissent pas avec ce même anticorps. En conséquence, la séquence nucléique introduite dans les clones phagiques sélectionnés doit correspondre à un pentadécapeptide qui permet la reconnaissance du phage par les anticorps 1E1E7 anti-P30.

### Exemple 2 : Détermination de la séquence des clones phagiques sélectionnés et analyse immunologique desdits clones par la technique ELISA.

L'ADN des phages correspondant aux 30 clones choisis a été préparé selon la méthode décrite par Sambrook et al., 1989. Molecular Cloning : A Laboratory Manual, 2nd Edition, Cold Spring Harbor Laboratory, Cold Spring Harbor.

Le séquençage de ces clones a été effectué suivant le protocole du fournisseur (United States Biochemicals) avec l'enzyme Séquénase (nom commercial) en utilisant une amorce de 18 bases (5'-TGAATTTTCTGTATGAGG-3'). Cette amorce est complémentaire des nucléotides 1663-1680 du gène codant pour la protéine PIII native du phage.

Les séquences nucléiques obtenues, lorsqu'elles sont converties en séquences d'acides aminés, indiquent que la séquence peptidique Trp-His-Trp-Arg-His-Arg-Ile-Pro-Leu-Gln-Leu-Ala-Ala-Gly-Arg est retrouvée un grand nombre de fois dans les clones sélectionnés. En effet, comme le montre le tableau suivant, sur les 30 clones séquencés, onze d'entre eux correspondent à cette séquence.

| Séquence des clones de phages | Nombre de séquences identiques |
|---|---|
| WHWRHRIPLQLAAGR | 11 |
| RHWRHRKPLQLATGR | 1 |
| LAFHRFNLSRPLQRD | 4 |
| THSHQWRHHQFPAPT | 3 |
| RRWVRYPVHLHSPIV | 5 |
| ARFPKELRGSVRSAH | 1 |
| GSWVLRHSSVGFHFV | 1 |
| GSFHWFRGSRHVVVH | 1 |
| SWRFFHSGMPRVSRS | 1 |
| GSPHRYRGARHVAVD | 1 |
| WKALFSHSYRSSGVP | 1 |

L'importance de ce motif a ensuite été analysée par test immunoenzymatique, en réalisant un test ELISA pour chacun des 30 clones phagiques sélectionnés. Néanmoins, il faut noter qu'après l'étape de purification, les clones à tester ne présentent pas tous la même concentration en phage et que ces différences peuvent entrainer des variations aspécifiques des mesures de Densité Optique lors du test ELISA. Afin de pallier ce problème, la fixation des phages sur les plaques d'ELISA a été standardisée par l'utilisation d'anticorps anti-phages M13 ajoutés à une concentration saturante fixe.

Ainsi, dans un premier temps, 100µl d'anticorps anti-phages M13 dilués au 1/1000 dans du NaHCO₃ 0,1M pH 8,6 (commercialisé par Prinn, Inc. Boulder, CO 80303) sont fixés une nuit dans les puits de plaques de microtitration Nunc maxisorb (nom commercial). Après deux lavages avec du TBS/Tween 0,05%, 100 µl des différents clones de phage purifiés sont ensuite incubés pendant deux heures à 37°C dans les puits. La saturation des sites aspécifiques est réalisée à l'aide de TBS contenant 1% de BSA pendant deux heures à 37°C. Après trois lavages avec du TBS/Tween 0,05%, 100µl de l'anticorps monoclonal anti-P30 1E1E7, à la concentration de 3nM, 4nM ou 6nM, sont ajoutés et incubés toute la nuit à 4°C. Après quatre lavages au TBS/Tween 0,05%, 100µl de conjugué anti-souris marqué à la peroxydase (commercialisé par Jackson Immuno Research Laboratories Inc.) sont ajoutés avant une incubation pendant une heure à 37°C. La réaction enzymatique de révélation est effectuée en ajoutant 100µl d'une solution H₂O₂/ ortho-phénylène-diamine (OPD) et en incubant l'échantillon 30 minutes à température ambiante. La réaction de coloration est interrompue par l'addition de 50µl d'acide sulfurique 1,8N. La densité optique est mesurée sur un lecteur de plaques BioMérieux à 492nm.

Ces résultats obtenus en ELISA confirment les résultats des séquençages précédents car les clones phagiques pour lesquels on observe les meilleures réponses en ELISA (DO variant entre 0,269 et 0,274 pour une concentration en anticorps monoclonal 1E1E7 de 3nM) possèdent une séquence nucléique correspondant au pentadécapeptide Trp-His-Trp-Arg-His-Arg-Ile-Pro-Leu-Gln-Leu-Ala-Ala-Gly-Arg. La séquence des clones pour lesquel on observe une réponse négative ne possède aucune homologie avec cette séquence. En outre, les résultats montrent que la réponse immunoenzymatique est d'autant plus élevée que la concentration en anticorps monoclonal 1E1E7 présent dans le test augmente.

### Exemple 3 : Inhibition par la protéine P30 native de la réponse ELISA obtenue avec le clone phagique identifié

Un test ELISA par compétition a été effectué, afin de déterminer si la réponse obtenue avec le clone phagique identifié est inhibée par une pré-incubation de l'anticorps monoclonal anti-P30 1E1E7 avec la protéine P30.

Le test ELISA est réalisé comme décrit dans l'exemple 2 avec les modifications suivantes: l'anticorps monoclonal 1E1E7 (à la concentration de 6nM) est pré-incubé avec différentes concentrations de protéine P30 pendant 20 minutes à 37°C, puis le mélange est ajouté dans les puits de la plaque ELISA dans lesquels sont fixés les phages correspondant au pentadécapeptide Trp-His-Trp-Arg-His-Arg-Ile-Pro-Leu-Gln-Leu-Ala-Ala-Gly-Arg.

Les résultats obtenus montrent que la réponse en ELISA est inhibée de 45% pour une concentration en protéine P30 de 35ng/ml. Cette inhibition atteint 80% avec une concentration en protéine P30 de 3,5µg/ml.

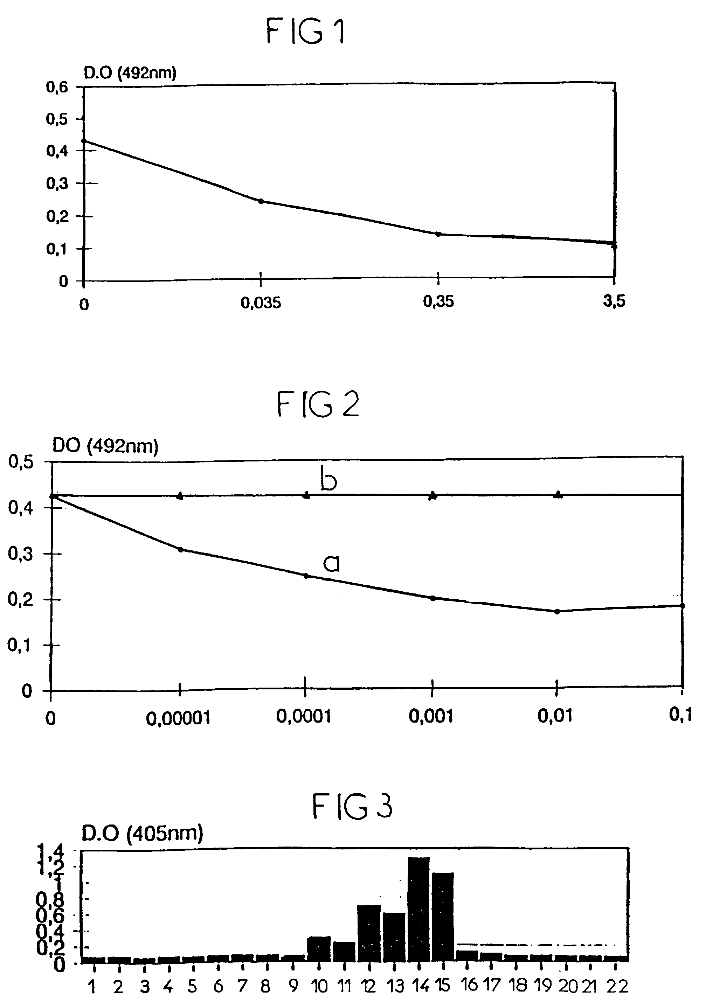

Les résultats obtenus (figure 1) sont standardisés par l'utilisation des anticorps anti-M13 comme décrit précédemment. La diminution de la réponse immunoenzymatique observée met clairement en évidence que le clone phagique identifié est reconnu par les mêmes anticorps capables de reconnaitre la protéine P30. Le clone phagique identifié, et notamment le pentadécapeptide Trp-His-Trp-Arg-His-Arg-Ile-Pro-Leu-Gln-Leu-Ala-Ala-Gly-Arg, est donc un compétiteur de la protéine P30 native, capable de se substituer à ladite protéine P30.

### Exemple 4 : Spécificité des anticorps humains anti-Toxoplasma gondii pour la séquence Trp-His-Trp-Arg-His-Arg-Ile-Pro-Leu-Gln-Leu-Ala-Ala-Gly-Arg portée par le clone phagique identifié

L'anticorps monoclonal 1E1E7 reconnait un site immunodominant sur la protéine P30. On a cherché à savoir si le pentadécapeptide Trp-His-Trp-Arg-His-Arg-Ile-Pro-Leu-Gln-Leu-Ala-Ala-Gly-Arg est également reconnu par les anticorps humains présents dans les séra de patient infecté par *Toxoplasma gondii.*

Le test ELISA est réalisé comme décrit dans l'exemple 2 avec les modifications suivantes : 100µl de différentes dilutions de sérum humain de patient infecté par *Toxoplasma gondii* (sérum positif - Référencé G8) sont mis en contact avec le clone phagique identifié, immobilisé comme décrit dans l'exemple 2, pendant 30 minutes à 37°C. Après deux lavages au TBS/Tween 0,05%, 100µl d'anticorps monoclonal 1E1E7 à la concentration de 6nM sont ajoutés et incubés durant une nuit à 4°C. Après quatre lavages au TBS/Tween, 100µl de conjugué anti-souris marqué à la peroxydase sont ajoutés puis incubés pendant une heure à 37°C. La réaction enzymatique de révélation est effectuée en ajoutant 100µl d'une solution H₂O₂/ ortho-phenylène-diamine (OPD) et en incubant l'échantillon 30 minutes à température ambiante. La réaction de coloration est interrompue par l'addition de 50µl d'acide sulfurique 1,8N. La densité optique est mesurée sur un lecteur de plaque BioMérieux à 492nm. Par ailleurs, un contrôle a été réalisé, dans les mêmes conditions réactionnelles mais avec un sérum humain non infecté par *Toxoplasma gondii* (sérum négatif), afin de s'assurer que le clone phagique identifié réagit de manière spécifique avec le sérum humain de patient infecté par *Toxoplasma gondii.*

Les résultats (figure 2) montrent que lorsque le sérum humain positif est dilué au 1/100 000, on observe une inhibition de 27% de la fixation de l'anticorps monoclonal 1E1E7 et qu'à partir de la dilution 1/100 du sérum humain, l'inhibition est maximale et atteint 60%.

Cet exemple montre donc que les phages renfermant la séquence Trp-His-Trp-Arg-His-Arg-Ile-Pro-Leu-Gln-Leu-Ala-Ala-Gly-Arg sont bien reconnus par les anticorps présents dans les sera des malades infectés par *Toxoplasma gondii.*

### Exemple 5 : Synthèses multiples de pentadécapeptides chevauchants et analyse immunologique desdits peptides par la technique ELISA.

Ayant obtenu des clones de phages qui donnent des réponses positives et spécifiques en immunoanalyse, nous avons cherché à déterminer le motif minimum reconnu spécifiquement par l'anticorps monoclonal 1E1E7 dans la séquence Trp-His-Trp-Arg-His-Arg-Ile-Pro-Leu-Gln-Leu-Ala-Ala-Gly-Arg.

Pour cela, nous avons effectué la synthèse chimique de peptides chevauchants qui couvrent cette séquence suivant les technologies développées d'une part par Berg et al. (1989, J. Ann. Chem. Soc., 111, 8024-8026) et commercialisée par Cambridge Research Biochemicals (nom commercial : Spotscan) et d'autre part par Geysen et al. (1987, J. of Immunological Methods, 102, 259-274) et commercialisée par Chiron (nom commercial : Pepscan) qui s'appuient l'une comme l'autre sur la synthèse simultanée d'un grand nombre de peptides et sur leur analyse par ELISA.

Ces méthodes permettent la synthèse de peptides chevauchants reproduisant une séquence déterminée.

Selon la technique "Pepscan", la synthèse est réalisée avec des acides aminés non estérifiés, protégés par un groupement FMOC (Nova Biochem). Les esters activés d'acides aminés sont formés directement sur une "couronne" au cours de la réaction de couplage. Après déprotection des acides aminés par une solution à 20% de pipéridine dans du N,N-diméthylformamide (DMF), le premier acide aminé est couplé à la "couronne" à la concentration de 30 ou 60nM. Les réactions de couplage utilisent de l'hexafluorophosphate de benzotriazol-lyl-oxy-tris(diméthylamine) phosphonium (BOP) comme agent couplant, de l'hydroxybenzotriazole (HOBT) et du N,N diisopropyléthylamine (DIEA) pour activer la réaction. Les acides aminés solubilisés dans du N-Méthyl-pyrrolidone (NMP) sont répartis dans les différents puits de la plaque ELISA et mis au contact des "couronnes" une nuit à température ambiante. Les peptides sont synthétisés grâce à des cycles successifs de déprotection par la pipéridine, lavage au DMF et addition d'un acide aminé par jour. Après ajout du dernier acide aminé, le groupement aminé terminal est acétylé par un mélange de DMF:anhydride acétique:triéthylamine (5:2:1, v/v/v) et les groupements protecteurs de la chaine latérale sont enlevés à l'aide d'un mélange d'acide trifluoroacétique:eau:phénol: éthanedithiol:thioanisole (85:4:5:2:4, v:v:w:v:v). L'immunoréactivité des peptides ainsi couplés à la surface des "couronnes" peut alors être testée par ELISA.

Selon la technique "Spotscan", la synthèse des peptides est réalisée sur des membranes de cellulose (Cambridge Research Biochemicals). Le principe de la méthode est le même que celui du Pepscan. Les acides aminés estérifiés sont solubilisés dans du N-méthyl pyrrolidone (NMP) à la concentration de 300nM et 0,9 µl sont déposés au niveau de taches de dépôt de bleu de bromophénol. Après une incubation de 15 minutes, un nouveau dépôt d'acides aminés est réalisé suivi d'une autre incubation de 15 minutes. Si le couplage entre les deux acides aminés s'est effectué correctement, on observe un changement de coloration (passage du bleu au vert-jaune). Après trois lavages dans du DMF, une étape d'acétylation est réalisée par de l'anhydride acétique. Puis les groupements aminés terminaux des peptides en cours de synthèse sont déprotégés par de la pipéridine 20% dans le DMF. Les taches de dépôt sont recolorées par une solution de bleu de bromophénol à 1% dans le DMF, lavées trois fois au méthanol et séchées. L'ensemble de ces opérations constitue un cycle d'addition d'un acide aminé et ce cycle est répété jusqu'à la fin de la synthèse. Lorsque tous les acides aminés ont été ajoutés, le groupement NH₂-terminal du dernier acide aminé est déprotégé par de la pipéridine à 20% dans le DMF et acétylé par de l'anhydride acétique. Les groupements protecteurs de la chaine latérale sont enlevés par un mélange dichlorométhane:acide trifluoroacétique:triisobutylsilane (5ml:5ml:250µl). L'immunoréactivité des peptides ainsi synthétisés peut alors être testée par ELISA

Afin de reproduire en totalité le pentadécapeptide inclus dans le clone phagique isolé et de déterminer le rôle éventuel des acides aminés contigus appartenant à la séquence de la protéine PIII du phage (voir Exemple 1), la synthèse chimique de pentadécapeptides chevauchants, qui couvrent la séquence Phe-Tyr-Ser-His-Ser-Ala-Asp-Gly-Ala-Trp-His-Trp-Arg-His-Arg-Ile-Pro-Leu-Gln-Leu-Ala-Ala-Gly-Arg-Gly-Ala-Ala-Gly-Ala-Glu-Thr-Val-Glu-Ser-Cys-Leu-Ala a été réalisée sur membrane de cellulose activée ("Spotscan").

Après synthèse des différents pentadécapeptides, la membrane est rincée avec du méthanol, lavée dans du TBS (Tris 0,1M pH7,2) puis incubée toute la nuit à température ambiante dans du tampon de saturation (commercialisé par Cambridge Research Biochemicals). Après plusieurs lavages avec du TBS-T (Tris 0,1M pH7,2 - 0,05% Tween 20), la membrane est mise en contact d'une solution d'anticorps monoclonal anti-P30 1E1E7 (60nM) et incubée 4 heures à température ambiante. Après trois lavages avec du TBS-T, le conjugué anti-immunoglobines de souris marqué à la β-galactosidase (commercialisé par Cambridge Research Biochemicals) est ajouté à une dilution au 1/200 et l'ensemble est incubé deux heures à température ambiante. Après plusieurs lavages de la membrane par du TBS-Tween 0,05% et du PBS, l'immunoréactivité au niveau des différentes taches est révélée par l'addition d'une solution de substrat (5-bromo-4-chloro-3-indoyl-β-D-galactopyranoside dans du PBS contenant du chlorure de magnésium et du ferricyanure de potassium) et incubation pendant 10 à 40 minutes. La coloration des tâches est estimée qualitativement.

L'analyse séquentielle des résultats obtenus montre que l'on obtient une première coloration avec la séquence Ser-Ala-Asp-Gly-Ala-Trp-His-Trp-Arg-His-Arg-Ile-Pro-Leu-Gln. Puis, la coloration bleue augmente progressivement lorsqu'on avance dans la séquence du phage pour atteindre un maximum avec les séquences His-Arg-Ile-Pro-Leu-Gln-Leu-Ala-Ala-Gly-Arg-Gly-Ala-Ala-Gly (SEQ ID N09) et Arg-Ile-Pro-Leu-Gln-Leu-Ala-Ala-Gly-Arg-Gly-Ala-Ala-Gly-Ala. Une très légère coloration est encore visible pour la séquence Ile-Pro-Leu-Gln-Leu-Ala-Ala-Gly-Arg-Gly-Ala-Ala-Gly-Ala-Glu mais elle disparait complètement à partir de la séquence Pro-Leu-Gln-Leu-Ala-Ala-Gly-Arg-Gly-Ala-Ala-Gly-Ala-Glu-Thr.

Ces résultats ont par ailleurs été confirmés par la synthèse des mêmes peptides par la technique dite "Pepscan" qui contrairement à la technique dite "Spotscan" permet de quantifier le niveau de réponse en ELISA obtenu pour chaque peptide.

Les "couronnes" portant les pentadécapeptides, synthétisés comme décrit précédemment dans des plaques de microtitration, sont saturées pendant une heure à l'aide d'une solution contenant 1% d'ovalbumine, 1% de sérum albumine bovine dans du PBS-T (tampon phosphate pH 7,2 contenant 0,1% de Tween 20). Après une incubation d'une nuit à 4°C avec 150 µl d'anticorps monoclonal 1E1E7 dilué au 1/100 (60nM), et quatre lavages avec du PBS-T, les puits de la plaque sont incubés pendant une heure avec du conjugué anti-souris marqué à la peroxydase dilué au 1/1000 (commercialisé par Jackson Immuno Research Laboratories Inc.). Suite à une série de quatre lavages avec du PBS-T, les puits de la plaque sont incubés avec le substrat ABTS (0,5 mg/ml azino-di-3-éthylbenzothiazo-line-6-sulphonate dans du tampon citrate pH 4,0 contenant 0,03% de peroxyde d'hydrogène). Les mesures d'absorbance sont effectuées à 405nm sur un lecteur de plaque ELISA (BioMérieux).

Par ailleurs, deux pentadécapeptides contenant les séquences Pro-Leu-Ala-Gln et Gly-Leu-Ala-Gln ont été synthétisés comme contrôles positif et négatif respectivement, et ont été testés en utilisant un anticorps monoclonal anti-(Pro-Leu-Ala-Gln).

Ces résultats (figure 3) confirment les résultats obtenus lors de la sélection du clone phagique (exemple 1) renfermant une séquence nucléique codant pour le pentadécapeptide Trp-His-Trp-Arg-His-Arg-Ile-Pro-Leu-Gln-Leu-Ala-Ala-Gly-Arg. Par ailleurs, ils montrent que les premiers acides aminés Trp-His-Trp-Arg à l'extrémité NH₂ ne sont pas nécessaires à la reconnaissance du peptide par les anticorps monoclonaux anti-P30 et que par contre l'addition de la séquence Gly-Ala-Ala-Gly à l'extrémité COOH du peptide permet d'améliorer cette réactivité. Enfin, ces résultats montrent qu'un peptide isolé, dont une suite de 11 acides aminés contigus ne présente pas plus de 36% d'homologie avec la séquence de la protéine P30 native, est capable d'être reconnu par des anticorps spécifiques de ladite protéine.

### Exemple 6 : Substitutions d'acides aminés dans le peptide His-Arg-Ile-Pro-Leu-Gln-Leu-Ala-Ala-Gly-Arg-Gly-Ala-Ala-Gly et analyse immunologique desdits peptides modifiés par la technique ELISA.

Afin de déterminer dans quelle mesure la nature des différents acides aminés constituant le peptide His-Arg-Ile-Pro-Leu-Gln-Leu-Ala-Ala-Gly-Arg-Gly-Ala-Ala-Gly (SEQ ID N09) peut varier sans affecter les propriétés antigéniques dudit peptide à l'égard des anticorps anti-P30, des essais de substitutions ponctuels ont été effectués.

Les peptides ont été synthétisés selon la méthode décrite dans l'exemple précédent, dite "Pepscan", en substituant sur le peptide chaque acide aminé à une position donnée par les dix-neuf autres. Ces peptides ont ensuite été testés par la technique ELISA comme décrit précédemment. L'importance de chaque résidu peut ainsi être examinée et un peptide regroupant les meilleurs acides aminés à chaque position peut être ensuite synthétisé.

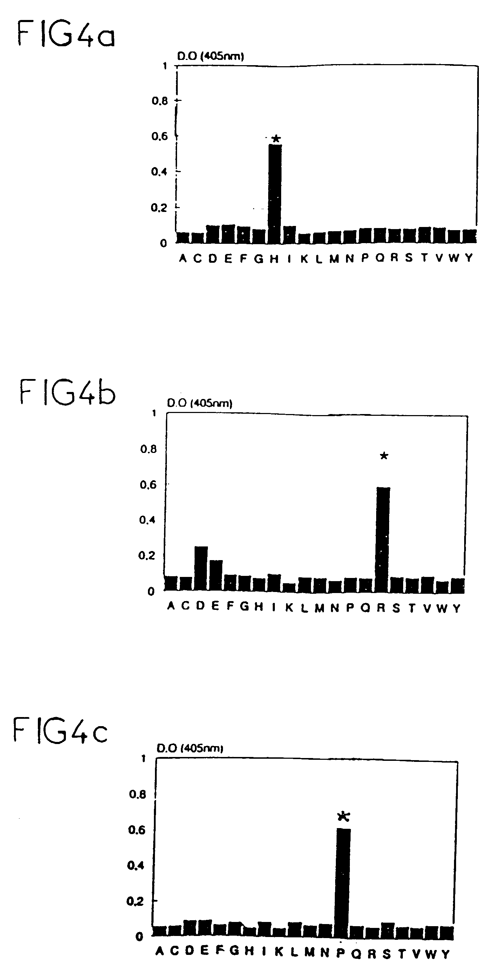

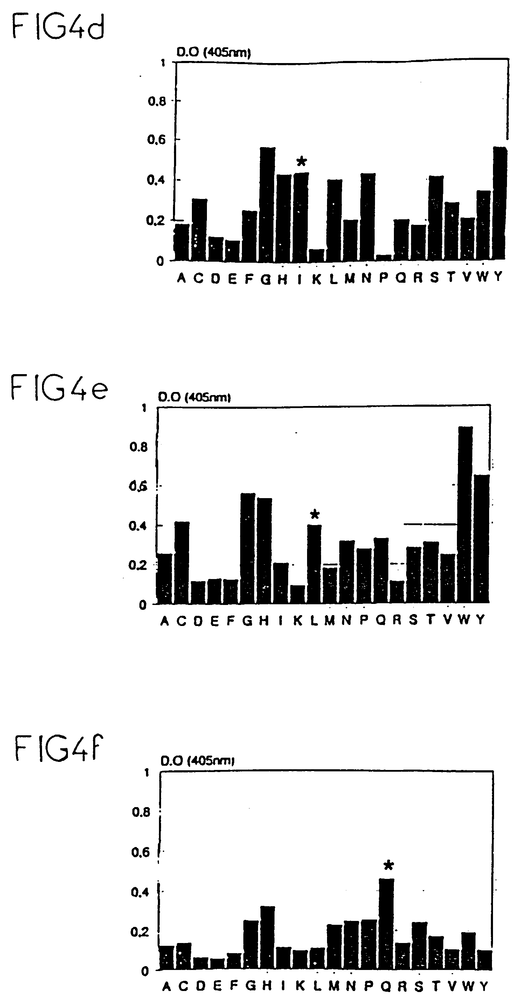

Les résultats obtenus (figures 4A à 4F) montrent que l'Histidine en position 1, l'Arginine en position 2, la Proline en position 4 et la Glutamine en position 6 semblent jouer un rôle important dans la reconnaissance par l'anticorps monoclonal anti-P30 1E1E7. Ces acides aminés donnent les signaux les plus élevés et doivent donc être conservés dans le peptide final.

Les résultats obtenus montrent également que lorsque l'Isoleucine en position 3 est remplacée par :
- la Glycine ou la Tyrosine, on observe une meilleure réponse,
- l'Histidine, la Leucine, l'Asparagine ou la Sérine, on observe une réponse équivalente à celle observée en présence de l'Isoleucine,
- la Proline, la Lysine, l'Acide Aspartique ou l'Acide Glutamique, on observe une disparition totale de la reconnaissance par l'anticorps monoclonal.

Les résultats montrent en outre que lorsque la Leucine en position 5 est remplacée par :
- les acides aminés aromatiques Tyrosine et Tryptophane, on obtient une très nette augmentation de la réponse ELISA,
- la Glycine et l'Histidine on obtient également une augmentation de la réponse avec les anticorps,
- la Cystéine, on observe une réponse équivalente à celle observée en présence de la Leucine,
- la Lysine, de l'Arginine, la Phénylalanine, l'Acide Aspartique et l'Acide Glutamique, on obtient une forte diminution de la réponse.

Pour les autres positions testées (la Leucine en position 7, l'Alanine en position 8 et en position 9, la Glycine en position 10 et l'Arginine en position 11), les résultats, obtenus par leur substitution par les 19 autres acides aminés, ne sont pas significativement différents de ceux observés avec le peptide de référence His-Arg-Ile-Pro-Leu-Gln-Leu-Ala-Ala-Gly-Arg-Gly-Ala-Ala-Gly.

### Exemple 7 : Synthèse chimique de deux entadécapeptides et test ELISA avec des sera humains Positifs.

Selon les indications obtenues à partir des exemples précédents, les pentadécapaptides His-Arg-Ile-Pro-Leu-Gln-Leu-Ala-Ala-Gly-Arg-Gly-Ala-Ala-Gly (SEQ ID N09) et His-Arg-Ile-Pro-Trp-Gln-Leu-Ala-Ala-Gly-Arg-Gly-Ala-Ala-Gly (SEQ ID N010) ont été synthétisés selon la méthode décrite dans la demande de brevet n° EP 93420183.

Ces peptides ont ensuites été testés en ELISA vis-à-vis de leur réactivité à l'égard de plusieurs séra de patients infectés par *Toxoplasma gondii.*

Les puits d'une plaque de microtitration Maxisorb (nom commercial, Nunc) sont saturés par 100 µl d'une solution de HCO₃Na 50mM pH 9,6 contenant 10 µg/ml de peptide à tester durant 2 heures à 37°C. La plaque est lavée avec du tampon PBS/ Tween 20 0,05%. Les puits sont ensuite saturés par 100 µl de tampon de lavage additionné de 1% d'albumine de sérum bovin pendant 1 heure à 37°C puis lavés comme décrit précédemment.

Les sera à analyser sont dilués au 1/200 et au 1/1000 dans le tampon de saturation et incubés 2 heures à 37°C. Après lavage, une solution de conjugué anticorps de chèvre anti-immunoglobuline humaine marqué à la péroxydase (commercialisé par Jackson Immuno Research Laboratories Inc.) est ajoutée à la dilution de 1/10 000 et les plaques sont incubées 1 heure à 37°C. Après lavages, le substrat ortho-phenylène-diamine (OPD) est ajouté et la réaction est arrêtée après 10 minutes par l'addition de 50 µl de H₂SO₄. La densité optique est lue à 492 nm.

Les résultats obtenus montrent que ces deux peptides ne réagissent pas du tout avec les séra négatifs et qu'ils sont spécifiquement reconnus par les séra de patients infectés par *Toxoplasma gondii.*

(1) INFORMATIONS GENERALES:

(i) DEPOSANT: BIO MERIEUX

(ii) TITRE DE L'INVENTION: Polypeptides mimotopes de
Toxoplasma gondii et applications.

(iii) NOMBRE DE SEQUENCES: 18

(iv) ADRESSE DE CORRESPONDANCE:
(A) NOM: Cabinet GERMAIN & MAUREAU
(B) RUE: 20 Bd Eugène Deruelle
(C) VILLE: LYON
(D) PAYS: FRANCE
(E) CODE POSTAL: 69003
(F) TELEPHONE: 72 60 28 90
(G) TELEFAX: 78 60 92 85

(v) FORME DECHIFFRABLE PAR ORDINATEUR:
(A) TYPE DE SUPPORT: disquette 3,5 pouces DS, HD
(B) ORDINATEUR: EPSON (compatible IBM)
(C) SYSTEME D'EXPLOITATION: DOS
(D) LOGICIEL: MICROSOFT WORD POUR WINDOWS

(vi) DEMANDE ACTUELLE:
(A) DATE DE LA DEMANDE:
(B) NUMERO DE LA DEMANDE:

(vii) DATE DE LA DEMANDE ANTERIEURE: 30.01.95
(A) NUMERO DE LA DEMANDE: 95 01297
(B) CLASSIFICATION: C07K

(viii) MANDATAIRE:
(A) NOM: CABINET GERMAIN & MAUREAU, Dominique GUERRE
(B) REFERENCE DOSSIER: MD/B 05 B 2172 EP

2) INFORMATIONS CONCERNANT SEQ ID NO:1:

i) CARACTERISTIQUES DE LA SEQUENCE
A) LONGUEUR : 336 acides aminés
B) TYPE : séquence d'acides aminés
ii) TYPE DE MOLECULE : protéine
iii) HYPOTHETIQUE : non
v) TYPE DE FRAGMENT :
vi) ORIGINE :
A) ORGANISME : protéine P30 de Toxoplasma gondii
B) SOUCHE :
xi) DESCRIPTION DE LA SEQUENCE : SEQ ID NO1
Met Ser Val Ser Leu His His Phe Ile Ile Ser Ser Gly Phe Leu Thr
1               5                   10                  15
Ser Met Phe Pro Lys Ala Val Arg Arg Ala Val Thr Ala Gly Val Phe
            20                  25                  30
Ala Ala Pro Thr Leu Met Ser Phe Leu Arg Cys Gly Val Met Ala Ser
        35                  40                  45
Asp Pro Pro Leu Val Ala Asn Gln Val Val Thr Cys Pro Asp Lys Lys
    50                  55                  60
Ser Thr Ala Ala Val Ile Leu Thr Pro Thr Glu Asn His Phe Thr Leu
65                  70                  75                  80
Lys Cys Pro Lys Thr Ala Leu Thr Glu Pro Pro Thr Leu Ala Tyr Ser
                85                  90                  95
Pro Asn Arg Gln Ile Cys Pro Ala Gly Thr Thr Ser Ser Cys Thr Ser
            100                 105                 110
Lys Ala Val Thr Leu Ser Ser Leu Ile Pro Glu Ala Glu Asp Ser Trp
        115                 120                 125
Trp Thr Gly Asp Ser Ala Ser Leu Asp Thr Ala Gly Ile Lys Leu Thr
    130                 135                 140
Val Pro Ile Glu Lys Phe Pro Val Thr Thr Gln Thr Phe Val Val Gly
145                 150                 155                 160
Cys Ile Lys Gly Asp Asp Ala Gln Ser Cys Met Val Thr Val Thr Val
                165                 170                 175
Gln Ala Arg Ala Ser Ser Val Val Asn Asn Val Ala Arg Cys Ser Tyr
            180                 185                 190

Gly Ala Asp Ser Thr Leu Gly Pro Val Lys Leu Ser Ala Glu Gly Pro
        195                 200                 205
Thr Thr Met Thr Leu Val Cys Gly Lys Asp Gly Val Lys Val Pro Gln
    210                 215                 220
Asp Asn Asn Gln Tyr Cys Ser Gly Thr Thr Leu Thr Gly Cys Asn Glu
225                 230                 235                 240
Lys Ser Phe Lys Asp Ile Leu Pro Lys Leu Thr Glu Asn Pro Trp Gln
                245                 250                 255
Gly Asn Ala Ser Ser Asp Lys Gly Ala Thr Leu Thr Ile Asn Lys Glu
            260                 265                 270
Ala Phe Pro Ala Glu Ser Lys Ser Val Ile Ile Gly Cys Thr Gly Gly
        275                 280                 285
Ser Pro Glu Lys His His Cys Thr Val Lys Leu Glu Phe Ala Gly Ala
    290                 295                 300
Ala Gly Ser Ala Lys Ser Ser Ala Gly Thr Ala Ser His Val Ser Ile
305                 310                 315                 320
Phe Ala Met Val Thr Gly Leu Ile Gly Ser Ile Ala Ala Cys Val Ala
                325                 330                 335

3) INFORMATION CONCERNANT SEQ ID NO2:
i) CARACTERISTIQUES DE LA SEQUENCE:
A) LONGUEUR : 6 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 3
D) AUTRES INFORMATIONS : Xaa signifie Gly ou His ou Leu ou
Asn ou Ser ou Tyr ou Ile
ix) CARACTERISTIQUES :
A) NOM/CLE : caractéristique particulière
B) EMPLACEMENT : 5
D) AUTRES INFORMATIONS : Xaa signifie Leu ou Trp ou Cys ou
Gly ou His ou Tyr
xi) DESCRIPTION DE LA SEQUENCE : SEQ ID NO2
His Arg Xaa Pro Xaa Gln
 1               5

3) INFORMATION CONCERNANT SEQ ID NO:3
i) CARACTERISTIQUES DE LA SEQUENCE:
A) LONGUEUR : 11 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 3
D) AUTRES INFORMATIONS : Xaa signifie Gly ou His ou Leu ou
Asn ou Ser ou Tyr ou Ile
ix) CARACTERISTIQUES :
A) NOM/CLE : caractéristique particulière
B) EMPLACEMENT : 5
D) AUTRES INFORMATIONS : Xaa signifie Leu ou Trp ou Cys ou
Gly ou His ou Tyr
ix) CARACTERISTIQUES :
A) NOM/CLE : caractéristique particulière
B) EMPLACEMENT : 7, 8, 9, 10, 11
D) AUTRES INFORMATIONS : Xaa signifie pour chaque
emplacement un acide aminé choisi parmi les 20 acides
aminés, indépendamment de l'acide aminé choisi pour les
autres emplacements.
xi) DESCRIPTION DE LA SEQUENCE : SEQ ID NO3
His Arg Xaa Pro Xaa Gln Xaa Xaa Xaa Xaa Xaa
1               5                   10

3) INFORMATION CONCERNANT SEQ ID NO:4
i) CARACTERISTIQUES DE LA SEQUENCE:
A) LONGUEUR : 15 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 3
D) AUTRES INFORMATIONS : Xaa signifie Gly ou His ou Leu ou
Asn ou Ser ou Tyr ou Ile
ix) CARACTERISTIQUES :
A) NOM/CLE : caractéristique particulière
B) EMPLACEMENT : 5
D) AUTRES INFORMATIONS : Xaa signifie Leu ou Trp ou Cys ou
Gly ou His ou Tyr
ix) CARACTERISTIQUES :
A) NOM/CLE : caractéristique particulière
B) EMPLACEMENT : 7, 8, 9, 10, 11
D) AUTRES INFORMATIONS : Xaa signifie pour chaque
emplacement un acide aminé choisi parmi les 20 acides
aminés, indépendamment de l'acide aminé choisi pour les
autres emplacements.
xi) DESCRIPTION DE LA SEQUENCE : SEQ ID NO4
His Arg Xaa Pro Xaa Gln Xaa Xaa Xaa Xaa Xaa Gly Ala Ala Gly
1               5                   10                  15

3) INFORMATION CONCERNANT SEQ ID N0:5
i) CARACTERISTIQUES DE LA SEQUENCE:
A) LONGUEUR : 11 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 3
D) AUTRES INFORMATIONS : Xaa signifie Gly ou His ou Leu ou
Asn ou Ser ou Tyr ou Ile
ix) CARACTERISTIQUES :
A) NOM/CLE : caractéristique particulière
B) EMPLACEMENT : 5
D) AUTRES INFORMATIONS : Xaa signifie Leu ou Trp ou Cys ou
Gly ou His ou Tyr
xi) DESCRIPTION DE LA SEQUENCE : SEQ ID N05
His Arg Xaa Pro Xaa Gln Leu Ala Ala Gly Arg
1               5                   10

3) INFORMATION CONCERNANT SEQ ID NO:6
i) CARACTERISTIQUES DE LA SEQUENCE:
A) LONGUEUR : 15 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 3
D) AUTRES INFORMATIONS : Xaa signifie Gly ou His ou Leu ou
Asn ou Ser ou Tyr ou Ile
ix) CARACTERISTIQUES :
A) NOM/CLE : caractéristique particulière
B) EMPLACEMENT : 5
D) AUTRES INFORMATIONS : Xaa signifie Leu ou Trp ou Cys ou
Gly ou His ou Tyr
xi) DESCRIPTION DE LA SEQUENCE : SEQ ID NO6
His Arg Xaa Pro Xaa Gln Leu Ala Ala Gly Arg Gly Ala Ala Gly
1               5                   10                  15

3) INFORMATION CONCERNANT SEQ ID N0:7
i) CARACTERISTIQUES DE LA SEQUENCE:
A) LONGUEUR : 11 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID N07
His Arg Ile Pro Leu Gln Leu Ala Ala Gly Arg
1               5                   10

3) INFORMATION CONCERNANT SEQ ID N0:8
i) CARACTERISTIQUES DE LA SEQUENCE:
A) LONGUEUR : 11 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID N08
His Arg Ile Pro Trp Gln Leu Ala Ala Gly Arg
1               5                   10

3) INFORMATION CONCERNANT SEQ ID NO:9
i) CARACTERISTIQUES DE LA SEQUENCE:
A) LONGUEUR : 15 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID NO9
His Arg Ile Pro Leu Gln Leu Ala Ala Gly Arg Gly Ala Ala Gly
1               5                   10                  15

3) INFORMATION CONCERNANT SEQ ID NO:10
i) CARACTERISTIQUES DE LA SEQUENCE:
A) LONGUEUR : 15 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID NO10
His Arg Ile Pro Trp Gln Leu Ala Ala Gly Arg Gly Ala Ala Gly
1               5                   10                  15

3) INFORMATION CONCERNANT SEQ ID NO:11
i) CARACTERISTIQUES DE LA SEQUENCE:
A) LONGUEUR : 33 paires de base
B) TYPE :
ii) TYPE DE MOLECULE : acide nucléique
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 6, 33
D) AUTRES INFORMATIONS : N signifie A, G, C ou T si M = C
dans le codon MGN, ou N signifie R si M = A dans le codon
MGN
ix) CARACTERISTIQUES :
A) NOM/CLE : caractéristique particulière
B) EMPLACEMENT : 15, 21
D) AUTRES INFORMATIONS : N signifie R si Y = T dans le
codon YTN, ou N signifie A, G, C ou T si Y = C dans le
codon YTN
xi) DESCRIPTION DE LA SEQUENCE : SEQ ID NO11
CAY MGN ATH CCN YTN CAR YTN GCN GCN GGN MGN 33

3) INFORMATION CONCERNANT SEQ ID NO:12
i) CARACTERISTIQUES DE LA SEQUENCE:
A) LONGUEUR : 33 paires de bases
B) TYPE : séquence de nucléotides
ii) TYPE DE MOLECULE : acide nucléique
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 6, 33
D) AUTRES INFORMATIONS : N signifie A, G, C ou T si M = C
dans le codon MGN, ou N signifie R si M = A dans le codon
MGN
ix) CARACTERISTIQUES :
A) NOM/CLE : caractéristique particulière
B) EMPLACEMENT : 21
D) AUTRES INFORMATIONS : N signifie R si Y = T dans le
codon YTN ou N signifie A, G, C ou T si Y = C dans le
codon YTN
xi) DESCRIPTION DE LA SEQUENCE : SEQ ID NO12
CAY MGN ATH CCN TGG CAR YTN GCN GCN GGN MGN 33

3) INFORMATION CONCERNANT SEQ ID N0:13
i) CARACTERISTIQUES DE LA SEQUENCE:
A) LONGUEUR : 45 paires de bases
B) TYPE : séquence de nucléotides
ii) TYPE DE MOLECULE : acide nucléique
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 6, 33
D) AUTRES INFORMATIONS : N signifie A, G, C ou T si M = C
dans le codon MGN ou N signifie R si M = A dans le codon
MGN
ix) CARACTERISTIQUES :
A) NOM/CLE : caractéristique particulière
B) EMPLACEMENT : 15, 21
D) AUTRES INFORMATIONS : N signifie R si Y = T dans le
codon YTN ou N signifie A, G, C ou T si Y = C dans le
codon YTN
xi) DESCRIPTION DE LA SEQUENCE : SEQ ID N013
CAY MGN ATH CCN YTN CAR YTN GCN GCN GGN MGN GGN GCN GCN GGN 45

3) INFORMATION CONCERNANT SEQ ID NO:14
i) CARACTERISTIQUES DE LA SEQUENCE:
A) LONGUEUR : 45 paires de bases
B) TYPE : séquence de nucléotides
ii) TYPE DE MOLECULE :
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 6, 33
D) AUTRES INFORMATIONS : N signifie A, G, C ou T si M = C
dans le codon MGN ou N signifie R si M = A dans le codon
MGN
ix) CARACTERISTIQUES :
A) NOM/CLE : caractéristique particulière
B) EMPLACEMENT : 21
D) AUTRES INFORMATIONS : N signifie R si Y = T dans le
codon YTN ou N signifie A, G, C ou T si Y = C dans le
codon YTN
xi) DESCRIPTION DE LA SEQUENCE : SEQ ID NO14
CAY MGN ATH CCN TGG CAR YTN GCN GCN GGN MGN GGN GCN GCN GGN 45

3) INFORMATION CONCERNANT SEQ ID NO:15
i) CARACTERISTIQUES DE LA SEQUENCE:
A) LONGUEUR : 54 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID NO15
Gly Glu Thr Gly Gly Arg Ser Arg Ser Ser Trp Asn Ala Arg Leu Ala
1               5                   10                  15
Arg Thr Gly Arg Asn Phe Arg Phe Tyr Asp Pro Ala Lys Val Lys Ser
            20                  25                  30
Leu Val Val Thr Asp Phe Ser Arg Leu Met Leu Arg Lys Ala Leu Glu
        35                  40                  45
Lys Lys Glu Ala Leu Arg
    50

3) INFORMATION CONCERNANT SEQ ID NO:16
i) CARACTERISTIQUES DE LA SEQUENCE:
A) LONGUEUR : 19 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID NO16
Thr Asp Phe Ser Arg Leu Met Leu Arg Lys Ala Leu Glu Lys Lys Glu
1               5                   10                  15
Ala Leu Arg

3) INFORMATION CONCERNANT SEQ ID NO:17
i) CARACTERISTIQUES DE LA SEQUENCE:
A) LONGUEUR : 19 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
ix) CARACTERISTIQUES :
A) NOM/CLE : caractéristique particulière
B) EMPLACEMENT : 1
D) AUTRES INFORMATIONS : Xaa signifie Acétylcystéine
xi) DESCRIPTION DE LA SEQUENCE : SEQ ID NO17
Xaa Asp Phe Ser Arg Leu Met Leu Arg Lys Ala Leu Glu Lys Lys Glu
1               5                   10                  15
Ala Leu Arg

3) INFORMATION CONCERNANT SEQ ID NO:18
i) CARACTERISTIQUES DE LA SEQUENCE:
A) LONGUEUR :85 acides aminés
B) TYPE : séquence d'acides aminés
ii) TYPE DE MOLECULE : peptide
iii) HYPOTHETIQUE : non
v) TYPE DE FRAGMENT :
vi) ORIGINE :
A) ORGANISME :
B) SOUCHE :
xi) DESCRIPTION DE LA SEQUENCE : SEQ ID NO18
Glu Gly Glu Thr Gly Gly Arg Ser Arg Ser Ser Trp Asn Ala Arg Leu
1               5                   10                  15
Ala Arg Lys Gln Ala Ile Asp Asp Gly Gln Gln Glu Glu Arg Gly Gln
            20                  25                  30
Leu Leu Gln Cys Arg Ser Ile Gly Ile Arg Pro Ala Gly Ser Leu Glu
        35                  40                  45
Ser Arg Arg Gly Ser Gln Glu Ser Gly Leu Arg Ala Ser Val Gly Phe
    50                  55                  60
Asp Ser Arg Val Leu Arg Cys Arg Lys Gly Gln Leu Arg Val Gly Glu
65                  70                  75                  80
Phe Arg Arg Pro Ala
                85

## Revendications

1. Polypeptide capable de réagir spécifiquement avec un anticorps anti protéine P30 de *Toxoplasma gondii,* et comprenant une séquence peptidique, dont toute suite de 6 acides aminés contigus présente au plus 67 % d'homologie avec la séquence peptidique identifiée par SEQ ID N01, ou comprenant une séquence dérivée de ladite séquence peptidique, à l'exclusion de tout polypeptide ayant une séquence peptidique antigénique vis à vis des anticorps anti P30 choisie parmi SEQ ID NO15, toute fraction de SEQ ID NO15, SEQ ID NO16, SEQ ID NO17, et SEQ ID NO18.

2. Polypeptide selon la revendication 1, comprenant une séquence peptidique, dont toute suite de 11 acides aminés contigus présente au plus 36 % d'homologie avec la séquence peptidique identifiée par SEQ ID N01, ou comprenant une séquence dérivée de ladite séquence peptidique.

3. Polypeptide selon la revendication 2, caractérisé en ce que sa séquence peptidique consiste en une séquence peptidique, dont toute suite de 11 acides aminés contigus présente au plus 36 % d'homologie avec la séquence peptidique SEQ ID N01, ou comprenant une séquence dérivée de ladite séquence peptidique.

4. Polypeptide capable de réagir spécifiquement avec un anticorps anti protéine P30 de *Toxoplasma gondii,* caractérisé en ce que sa séquence peptidique comprend SEQ ID N02, ou une séquence dérivée de ladite séquence peptidique.

5. Polypeptide selon la revendication 4, caractérisé en ce que sa séquence peptidique comprend une séquence choisie parmi SEQ ID N03, SEQ ID N04, et leurs séquences respectivement dérivées.

6. Polypeptide selon la revendication 5, caractérisé en ce que sa séquence peptidique comprend une séquence choisie parmi SEQ ID N05, SEQ ID N06, et leurs séquences respectivement dérivées.

7. Polypeptide selon la revendication 6, caractérisé en ce que sa séquence peptidique comprend une séquence peptidique choisie parmi SEQ ID N07, SEQ ID N08, SEQ ID N09, SEQ ID N010, et leurs séquences respectivement dérivées.

8. Polypeptide selon l'une quelconque des revendications 4 à 7, caractérisé en ce que sa séquence peptidique consiste en une séquence peptidique choisie parmi SEQ ID N02, SEQ ID N03, SEQ ID N04, SEQ ID N05, SEQ ID N06, SEQ ID N07, SEQ ID N08, SEQ ID N09, SEQ ID N010, et leurs séquences respectivement dérivées.

9. Réactif pour la détection d'une infection par *Toxoplasma gondii* caractérisé en ce qu'il comprend, à titre de substance réactive, un polypeptide selon l'une quelconque des revendications 1 à 8, ledit polypeptide étant éventuellement marqué.

10. Kit de détection d'une infection par *Toxoplama gondii,* caractérisé en ce qu'un réactif selon la revendication 9 est supporté par un support solide, immunologiquement compatible avec ledit réactif.

11. Procédé pour la détection, la séparation notamment purification, et/ou le dosage d'anticorps anti protéine P30 de *Toxoplama gondii* dans un échantillon de fluide biologique caractérisé en qu'on met en contact ledit échantillon avec un réactif selon la revendication 9, dans des conditions permettant une réaction immunologique, et en ce qu'on détecte, sépare et quantifie le complexe immun éventuellement formé.

12. Procédé pour le dosage de la protéine P30 de *Toxoplama gondii* dans un échantillon d'un fluide biologique, par une technique de compétition, caractérisé en qu'on met simultanément en contact ledit échantillon avec une quantité prédéterminée d'anticorps anti protéine P30, et une quantité prédéterminée d'un réactif selon la revendication 9, et on détermine la quantité de protéine P30 dans ledit échantillon par déduction à partir de la quantité mesurée du complexe immun formé entre le réactif et lesdits anticorps anti protéine P30.

13. Procédé pour le dosage de la protéine P30 de *Toxoplama gondii* dans un échantillon d'un fluide biologique, caractérisé en que dans un premier temps, on met en contact ledit échantillon avec une quantité prédéterminée d'anticorps anti protéine P30, dans un second temps, on ajoute une quantité prédéterminée d'un réactif selon la revendication 9, et on détermine la quantité de protéine P30 dans ledit échantillon par déduction à partir de la quantité mésurée du complexe immun formé entre le réactif et lesdits anticorps anti protéine P30.

14. Composition immunothérapeutique active, notamment préparation vaccinale, caractérisée en ce qu'elle comprend à titre de principe actif, un polypeptide selon l'une quelconque des revendications 1 à 8, ledit principe actif étant éventuellement conjugué avec un support immunologiquement adapté, et éventuellement un excipient pharmaceutiquement acceptable.

15. Polynucléotide comprenant une séquence nucléotidique codant pour un polypeptide selon l'une quelconque des revendications 1 à 8.

16. Polynucléotide selon la revendication 15, caractérisé en ce qu'il comprend une séquence nucléotidique choisie parmi SEQ ID N011, SEQ ID N012, SEQ ID N013, SEQ ID N014.

17. Cassette d'expression fonctionnelle permettant l'expression d'un polynucléotide selon la revendication 15 ou 16 et comprenant ce dernier.

18. Vecteur comprenant une cassette d'expression selon la revendication 17.

19. Matériel cellulaire eucaryote ou procaryote comprenant une cassette d'expression selon la revendication 17 ou un vecteur selon la revendication 18.
